# EUROPEAN PATENT APPLICATION

(11) **EP 4 707 713 A2**
(43) Date of publication of application: **11.03.2026**
(21) Application number: 26152057.1
(22) Date of filing: 22.11.2022
(51) Int. Cl.: F25D 31/00

(54) **COLD THERAPY SYSTEM AND HEAT EXCHANGE DEVICE THEREFOR**

(30) Priority: 24.11.2021 GB 202116884
(62) Divisional of application: 22817308.4
(71) Applicant: Cryoshower Limited, Swanage Dorset BH19 2PQ (GB)
(72) Inventor: Skivington, Dean, Swanage, BH19 2PQ (GB)
(74) Representative: Strachan, Victoria Jane

(57) **Abstract**

A cold therapy system comprising a housing 10 having an inlet 12 for receiving fluid from a source and an outlet 14 for delivering cooled fluid, the housing defining a chamber comprising or defining at least one conduit 20 for removably receiving a heat exchange device 30 comprising a body of ice, the conduit 20 being disposed in a fluid flow path between the inlet 12 and the outlet 14 such that, in use, the fluid flows from said inlet 12 over the outer circumference of said body of ice 30 before delivery to said outlet 14.

## Description

### Field of the Invention

This invention relates generally to a cold therapy system and to a heat exchange device that has particular, but not necessarily exclusive, utility in a cold therapy system.

### Background of the Invention

Localised application of ice packs, or other cooling means, has long been accepted as an effective way to treat joint and tissue injuries, to reduce swelling and inflammation and ease pain. On the basis that exposure to cold is known to reduce inflammation and swelling and ease sore muscles, many athletes use ice baths as a means to speed up recovery after physical exercise, and even cold showers are reported to reduce stress, increase alertness and stimulate a more robust immune response. However, freezing enough ice to cool a bath full of water sufficiently is a challenge in itself, and cold water straight from the mains supply is not usually sufficiently cold to effect significant physical therapy.

There is, therefore, a need for a cold therapy system that can efficiently cool and deliver a flow of fluid, received from a source and cooled to cold therapy temperatures (below 15°C) as it flows through the system, which could be installed and used in a user's home and even be connected to their mains water supply. Aspects of the present invention seek to address at least one or more of these issues.

### Summary of the Invention

In accordance with a first aspect of the invention, there is provided a cold therapy system comprising a housing having an inlet for receiving fluid from a source and an outlet for delivering cooled fluid, the housing defining a chamber comprising or defining at least one conduit for removably receiving, for use, a heat exchange device comprising a body of ice, the conduit being disposed in a fluid flow path between the inlet and the outlet such that, in use, the fluid flows from said inlet over the outer circumference of said body of ice before delivery to said outlet.

The heat exchange device may comprise a solid ice pack, which may be generally cylindrical with a circular or oval cross-section, although this is not necessarily essential, and cross-sections of other geometric shapes are envisaged in some embodiments, depending on the shape of the mould used to form the ice pack. Optionally, the heat exchange device may comprise a pair of solid (e.g. generally semi-cylindrical) ice packs arranged in said conduit with edges facing each other and a gap therebetween, and configured such that, in use, fluid flows through said gap (as well as over the outer circumferences of the ice packs).

The system may further comprise a removable, generally tubular sleeve in said conduit for receiving said ice pack; and, optionally, the removable sleeve may have apertures in its side wall.

In an exemplary embodiment, the heat exchange device may comprise a plurality of compartments filled with ice for use, the compartments being arranged in side-by-side configuration to form a substantially solid ice pack, in use.

In accordance with a second aspect of the invention, there is provided a heat exchange device for use in a cold therapy system substantially as described above, the heat exchange device comprising a solid ice pack having exposed ice over substantially all of its circumferential area and being configured to fit into said conduit for use.

According to another aspect of the invention, there is provided a re-usable mould for making a heat exchange device substantially as described above, the mould comprising a first mould portion defining a cavity therein, and a second mould portion comprising a cover shaped and configured to fit over and cover said cavity, and having one or more through-holes therein to allow said cavity to be filled with water before freezing.

In an embodiment, the cavity may have a generally semi-circular or semi-oval profile.

Optionally, the mould may further comprise an insert member configured to fit within said cavity, said insert member comprising an elongate spine having at least one external collar or fin thereon. The insert member, which is re-usable, may be removably located (or locatable) in said cavity.

In an embodiment, the re-usable mould may comprise a plurality of (e.g. semi-circular or semi-oval) collars or fins disposed in spaced-apart relation along the length of the spine, each collar or fin comprising a first substantially straight edge and remaining body portion, a portion of each collar or fin adjacent the respective straight edge extending beyond the surface of the spine on one side and the remaining body portion of each collar or fin extending beyond the surface of the spine on the opposite side thereof. For example, in an embodiment where the collars or fins are semi-circular or semi-oval in shape, they may comprise a first substantially straight edge that extends beyond the surface of the spine on one side and remaining, arcuate body portion that extends beyond the surface of the spine on the opposite side thereof.

In another embodiment, the re-usable mould may comprise an insert having a plurality of symmetrically-shaped collars or fins disposed substantially symmetrically along the length of a central spine, each collar or fin comprising a pair of plates of substantially half said symmetrical shape each pair of plates being disposed on opposite sides of said central spine, and in spaced-apart relation, such that a gap is defined between the adjacent edges of each pair of plates. In an example, each collar or fin may comprise of a pair of generally semi-circular or semi-oval plates disposed on opposing sides of the spine to form a generally circular or oval member having a diametric gap between the plates adjacent the spine. The gaps between the pairs of plates, collectively, form an elongate channel through the insert which, in use, defines a channel through the ice pack through which fluid can flow.

In yet another embodiment, the spine may comprise a generally tubular member having a plurality of circumferential fins (e.g. generally circular or semi-oval, although the present invention is not intended to be limited in this regard) disposed in spaced-apart relation thereon. In this case, the tubular spine may, optionally, be configured to receive an insert substantially as described above.

Optionally, apertures may be provided in the walls of the collars or fins and/or the spine of any of the inserts described above.

In accordance with another aspect of the invention, there is provided a re-usable mould for making a heat exchange device substantially as described above, comprising a plurality of compartments configured to receive a quantity of water therein for freezing, said compartments being arranged in side-by-side relation and pivotally coupled together at adjacent edges, the mould being configured to be moved, by pivoting said compartments relative to each other, from an open configuration in which said compartments can be filled with water and frozen, and a closed configuration in which said compartments form a three-dimensional ice pack having a circumferential side wall of ice.

In an embodiment, the compartments may be elongate and of substantially triangular cross-section, and wherein said compartments may be pivotally coupled together at adjacent longitudinal edges such that, when in said closed configuration, the apexes of said compartments are located adjacent the centre of the mould and define an elongate channel therebetween that extends along the longitudinal axis of the mould.

In an embodiment, the compartments may be elongate and of generally truncated triangular cross-section, and wherein the compartments may be pivotally coupled together at adjacent longitudinal edges such that, when in the cylindrical configuration, the truncated apexes of said compartments face the centre of the mould and together form a tubular channel therethrough. In this case, the tubular channel may shaped and configured to receive the re-usable mould substantially as described above when in the cylindrical configuration.

An aspect of the invention extends to a kit of parts comprising a cold therapy system substantially as described above and at least one re-usable mould substantially as described above.

Further, an aspect of the invention extends to an ice pack formed using a re-usable mould substantially as described above.

An aspect of the invention extends still further to the use of a re-usable heat exchange device substantially as described above in a cold therapy system substantially as described above.

It is to be understood that the terms generally "cylindrical" and semi-cylindrical used herein are intended to be interpreted broadly and purposively. They may refer to a geometric shape having a circular or oval cross-section, although it is to be understood that the cross-sectional shape could, in fact, comprise any closed, generally rounded shape. The side walls may be parallel, or they may be tapered to resemble a truncated cone. All of these, and other, variations are intended to be encompassed by the terms "generally cylindrical" and "generally semi-cylindrical".

These and other aspects of the present invention will become apparent from the following detailed description.

### Brief Description of the Drawings

Embodiments of the present invention will now be described, by way of examples only, and with reference to the accompanying drawings, in which:
Figure 1A is a schematic perspective view of the container section of a housing of a cold therapy system according to an exemplary embodiment of the present invention;
Figure 1B is a schematic perspective view of a lid of a housing of the cold therapy system of Figure 1A;
Figure 1C is a schematic perspective view of a cold therapy system according to an exemplary embodiment of the present invention, including the container section of Figure 1A and the lid of Figure 1B;
Figure 2A is a schematic perspective view of a in ice pack mould, in its open configuration, for creating an ice pack for use in a cold therapy system;
Figure 2B is a schematic cross-sectional view of the mould of Figure 2A;
Figure 2C is a schematic perspective view of the ice pack mould of Figure 2A removably mounted in a tray for ease of filling and transport;
Figure 3A is a schematic perspective view of the ice pack mould of Figure 2A when in the rolled configuration for use;
Figure 3B is a schematic cross-sectional view of the ice pack mould of Figure 3A;
Figure 4A is a schematic perspective view of an example ice pack for use in a cold therapy system;
Figure 4B is a schematic lateral cross-sectional view of the ice pack of Figure 5A;
Figure 5A is a schematic perspective view of an ice pack mould in its open configuration, for use in creating an ice pack for use in a cold therapy system;
Figure 5B is a schematic perspective view of the ice pack mould of Figure 6A, when in a closed configuration;
Figure 6 is a schematic perspective view of an insert for an ice pack mould for use in creating an ice pack for use in a cold therapy system;
Figure 7A is a schematic perspective view of an ice pack in use within a cold therapy system, illustrating the flow of fluid to be cooled in respect thereof;
Figure 7B is a schematic perspective view of the ice pack of Figure 8A when some of the ice thereon has melted during use;
Figure 8 is a schematic perspective view of a sleeve or sheath that can be removably mounted in a conduit of a cold therapy system for receiving therein an ice pack;
Figure 9 is a schematic perspective view of an insert for an ice pack mould for use in creating an ice pack for a cold therapy system;
Figure 10A is a schematic cross-sectional view of an example aperture of an ice pack mould insert or a sleeve or sheath for use in a cold therapy system;
Figure 10B is a schematic cross-sectional view of another example aperture of an ice pack mould insert or a sleeve or sheath for use in a cold therapy system; and
Figures 11A and 11B are schematic perspective views of an inner insert (Figure 12A) and an outer insert (Figure 12B) for an ice pack mould for use in creating an ice pack for use in a cold therapy system.

### Detailed Description

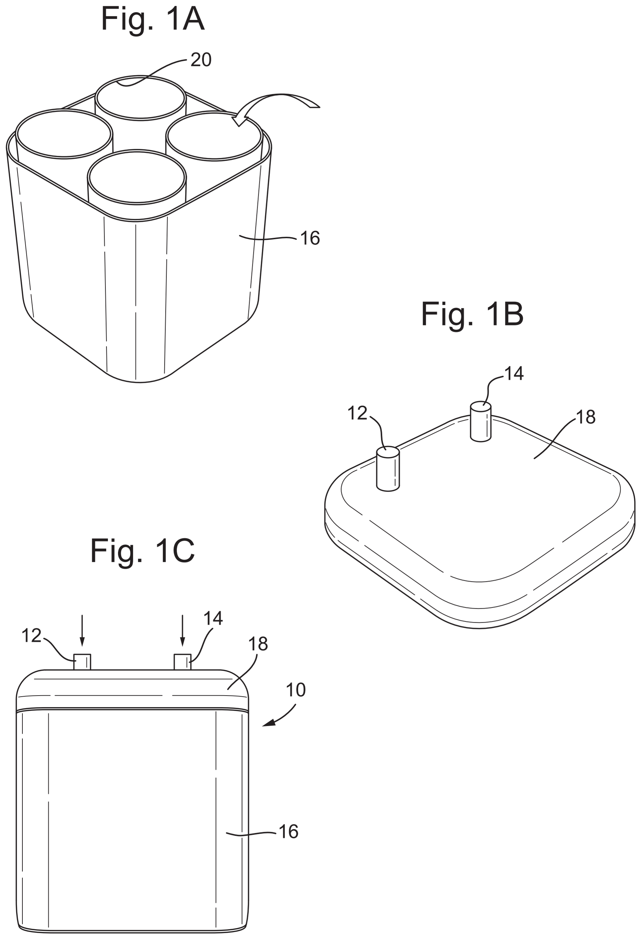

Referring first to Figures 1A, 1B and 1C of the drawings, a cold therapy system according to an exemplary embodiment of the present invention comprises a housing 10 having an inlet 12 and an outlet 14. The inlet 12 is configured to receive a flow of fluid, such as water for example, from a tap or mains water supply, although the present invention is not necessarily intended to be limited in this regard. The housing 10 comprises a container section 16 having an open end (see Figure 1A) and a lid 18 (see Figure 1B) configured to be removably fitted over the open end of the container section 16 for use. In this exemplary embodiment, the inlet 12 and the outlet 14 are both disposed in the lid 18, although the present invention is not necessarily intended to be limited in this regard. The housing 10 is beneficially formed of a rigid, thermally insulative material such as an organic plastic material, although the present invention is not limited in this regard and suitable materials will be apparent to a person skilled in the art.

Referring specifically to Figure 1A of the drawings, the container section 16 defines one or more, in this case four, open ended conduits 20, each configured to receive a re-usable heat exchange device (not shown in Figure 1A, and as will be described in more detail hereinafter). In use, fluid (for example cold water at or around a temperature of 18°C or more) enters the housing 10, via the inlet 12. As an example, cold water from a tap or mains supply may enter the housing 10, via the inlet 12, at a pressure of around 1bar (although this will vary greatly depending on the supply, and is not intended to be in any way limiting).

The pressure of the fluid flowing into the housing causes it to flow over, into and between the conduits 20, such that it flows over, between and through the ice packs contained therein. The fluid is thus cooled by the ice packs before delivery thereof to the outlet 14. The continuous flow of fluid through the housing helps to prevent it from being frozen and solidifying as it comes into contact with the ice packs as it flows from the inlet, through the housing to the outlet, and water (for example) temperature can be reduced by more than 5°C efficiently and effectively. The ice packs, as well as the number and configuration of the conduits 20 within the housing can be designed such that the fluid is distributed over the solid ice packs to optimise heat transfer, as will be described in more detail hereinafter. The degree of temperature reduction and/or the speed of cooling can be regulated depending on the number of conduits (and, therefore, ice packs) are provided in the housing. It is envisaged that smaller units could comprise a single conduit with a single ice pack within a housing having an inlet at one end and an outlet at the other, such that fluid flows over the ice pack in the housing as it flows from the inlet to the outlet. In alternative embodiments, two or more conduits may be provided. In the illustrated example, four conduits are provided, but it is envisaged that fewer than this may be adequate for some applications, and more can be provided for others, and the present invention is not necessarily intended to be limited in this regard. More generally, it will be understood that the cooling of the fluid (i.e. parameters such as degree of temperature reduction, amount of fluid cooled, time for which cooled fluid can be continuously delivered, rate of cooling, etc) can be carefully controlled by controlling various parameters and characteristics (supply fluid pressure, number of ice packs, configuration of ice packs, size of ice packs, etc). For example, simply altering the pressure at which the fluid is deliver to the system at the inlet 12 can alter the rate of flow of fluid through the housing which, in turn, could increase the degree of temperature reduction and/or the amount of time for which cooled fluid can be continuously delivered (before the ice packs need to be changed because they have melted). A valve and/or pump arrangement could be provided for this purpose, so that a user can change these parameters as required. The number and/or size of the ice packs can be varied, and their configuration within the housing can also be used to vary the degree and rate of cooling, as well as the amount of time cooled fluid can be delivered continuously. The configuration of the ice packs themselves can contribute greatly to the degradation (melting) of the ice while fluid is flowing over them, and, together with the internal design of the container section 16, the system can be designed to, in use, distribute fluid over the ice in the ice packs so as to optimise heat transfer.

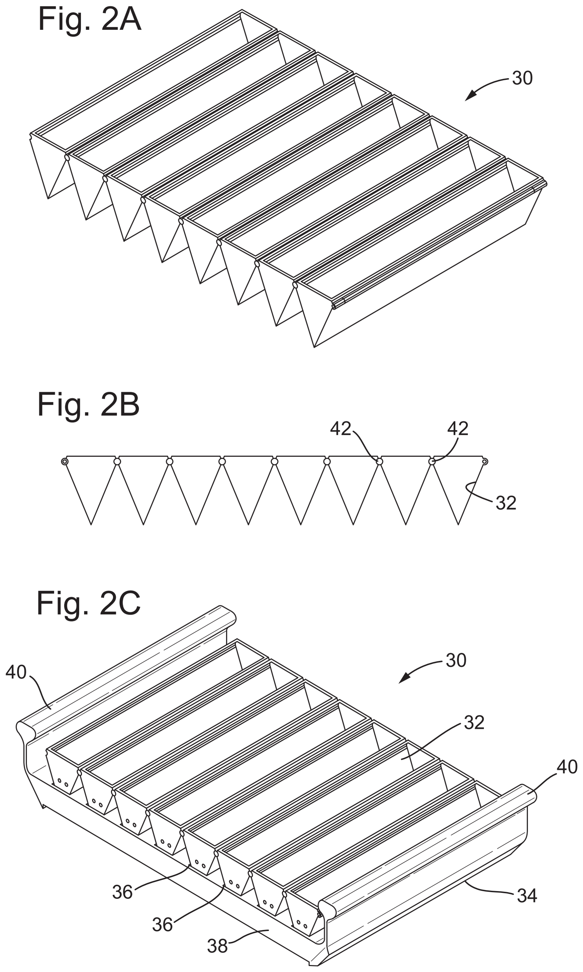

Referring now to Figures 2A, 2B and 2C of the drawings, there is illustrated schematically a re-usable ice pack mould 30 for use in creating an ice pack for use in a cold therapy system such as that described above. In Figure 2A, the ice pack mould 30 is illustrated in an extended, 'open' configuration. The ice pack mould 30 comprises an array of elongate compartments 32 arranged in side-by-side configuration, with the long edges thereof parallel to each other. Each compartment 32 is of generally triangular (lateral) cross-section having an apex and an opposing, open 'base' end. Each of the adjacent pairs of compartments 32 are pivotally connected together along their respective base edges (as shown schematically in Figure 2B of the drawings). In order to prepare an ice pack for use, the compartments 32 are filled with water (with the ice pack mould in the open configuration illustrated in Figure 2A) and the ice pack mould, thus filled, is placed into a freezer device until the water in the compartments 32 has frozen solid. A tray 34 (as illustrated in Figure 2C) may be provided on which the ice pack mould 30 can stand whilst it is being filled and frozen. The tray 34 comprises a series of elongate notches or grooves 36, of generally triangular (lateral) cross-section, arranged in parallel, spaced-apart relation along a base 38. A pair of handle members 40 may be provided at opposite respective ends of the base 38. In use, the ice pack mould 30 can be removably mounted (in the open configuration) on the tray 34 such that the apex of each compartment 32 is received within a respective groove 36 in the base 38. The compartments 32 can then be filled and the assembly conveniently transported, using the tray 34, to the freezer with reduced risk of spillage. The tray 34 offers a stable, flat base on which the ice pack mould 30 can sit in the freezer device, and also acts to prevent deformation of the ice pack mould 30 as the water in the compartments 32 freezes.

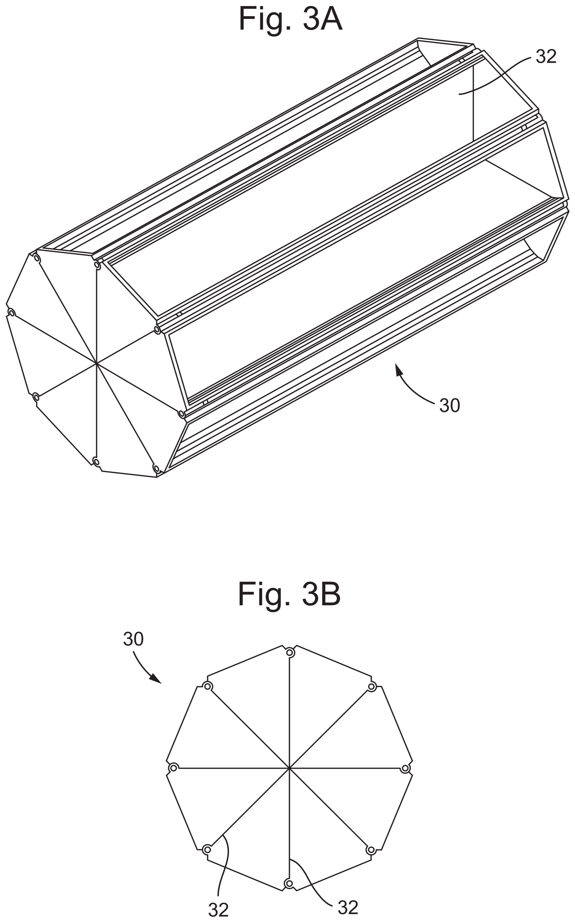

Referring now to Figures 3A and 3B of the drawings, once the water in the compartments 32 has frozen solid, the ice pack mould 30 can be removed from the tray 34 and 'rolled' into a generally cylindrical shape by pivoting the compartments relative to each other, such that the apexes of all of the compartments 32 extend along the centre of the cylinder and the open base ends thereof, and the solid surface of ice thereat, form its outer circumference (see Figure 3B). This is achieved by means of the pivotal connection (along the base edges of adjacent compartments), as referenced above.

It will be apparent to a person skilled in the art that there are numerous different ways in which the pivotal coupling between adjacent compartments can be achieved, and the present invention is not intended to be in any way limited in this regard.

Once the ice pack 30 has been thus prepared, and rolled into the cylindrical shape illustrated in Figure 3A, it can be inserted for use into one of the conduits 20 of a cold therapy system such as that illustrated schematically in Figures 1A, 1B and 1C. When all of the conduits 20 have respective ice packs 30 therein, the lid 18 can be sealed over the open end of the container section 16, and the inlet can be connected to a fluid (e.g. cold water) supply. The outlet 14 can be provided with, or connected to, a delivery system (not shown), e.g. a nozzle, spray, shower head, hose, etc. As fluid enters the housing 10 via the inlet 12, the fluid (under the supply pressure) is caused to flow through the conduits 20 and over and through the ice packs 30 housed therein. The configuration of the system and the ice packs is such that fluid will flow over the top of the ice packs, as well as over the side circumferential walls and also through small gaps between the ice pack compartments 32. Additionally, any ice in the ice pack 30 that melts during use will flow into the fluid flow path, further contributing to the cooling of the fluid. As a result, the cooling properties of each ice pack can be maximised (in terms of efficiency and the time for which it is effective) by maximising the surface area used to effect cooling, whilst minimising the physical space needed to house the heat exchange devices. The proximity of the ice-filled compartments to each other, during use, slows the degradation (melting) of the ice therein. In effect, this means that there is an opportunity for careful management of the heat exchange process, to enable flow and heat transfer for a defined amount of time, using passive re-usable heat exchange devices (ice packs) that take up minimal physical space and don't melt too quickly. The number, size and precise configuration of the ice packs used in the system can be varied to achieve such management, as can the rate of flow of fluid and the size and configuration of the container section 16 of the housing 10.

Furthermore, alternative ice pack designs are envisaged that could further optimise the heat transfer management aspect of exemplary cold therapy systems. These alternative ice packs could be interchangeable with those of a type described above, or they could offer alternative respective cold therapy systems that provide varying cold therapy characteristics. For example, an embodiment of the invention can be designed to create a reduction in water temperature by around 5°C for a period of time such as 8 - 10 minutes. However, it will be understood that these characteristics can vary depending on various parameters of the system. For example, the size and number of ice packs used, their configuration, and the materials used within the ice packs can all be varied to vary the characteristics of the resultant cold therapy system.

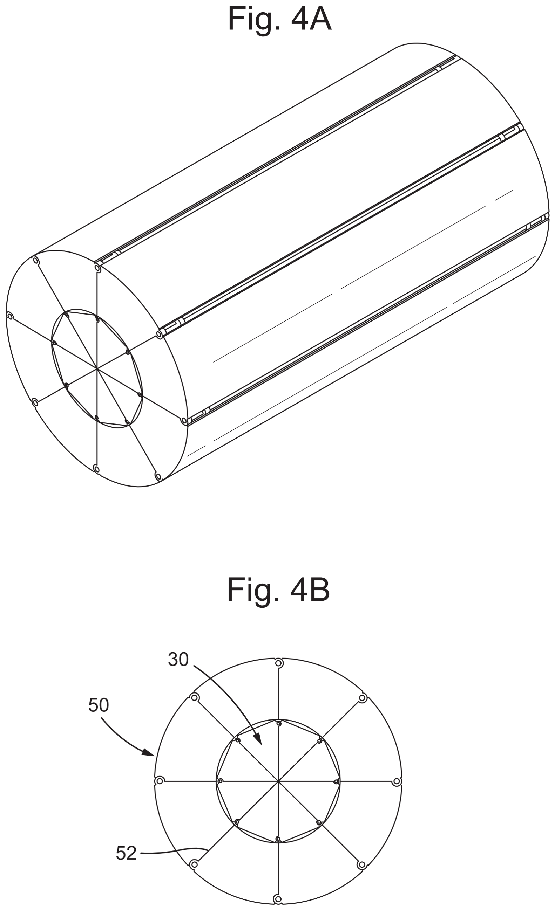

For example, referring to Figures 4A and 4B of the drawings, the ice pack could comprise could comprise a second ice tray 50 having the same number of compartments as the ice tray 30 described above (in this case, eight, but the invention is not necessarily limited in this regard), each compartment 52 having, in this case, a truncated triangular (lateral) cross-section, with the inner surface (opposite the open end) of each compartment being rounded and convex (when viewed from the perspective of the open end). The width of each compartment, at the convex inner surface, is substantially equal to that at the open end of each compartment 32 of the first ice tray 30, and the angle at which the side walls of each compartment 52 extend is substantially the same as that of the side walls of each compartment 32 of the first ice tray 30. Once again, adjacent compartments 52 of the second ice tray 50 are pivotally coupled together such that the ice tray 50 can be 'rolled' around the 'rolled up' first ice tray 30, leaving a small gap between the concave outer surface (i.e. the opposite side of the inner surface referenced above) of each compartment 52 and the open edge of the adjacent compartment 32 of the first ice tray 30. As such, a much larger ice pack can be provided, which stays frozen for longer and has a much larger circumferential heat exchange surface area, and also allows the flow of fluid through the channels provided by the above-mentioned small gaps between the first and second ice trays, thereby further increasing the surface area over which heat exchange (cooling) can occur. This may be embodied in a greater temperature reduction of the fluid and/or a longer period of operation and/or a greater body of fluid that can be cooled by an ice pack before it needs to be replaced.

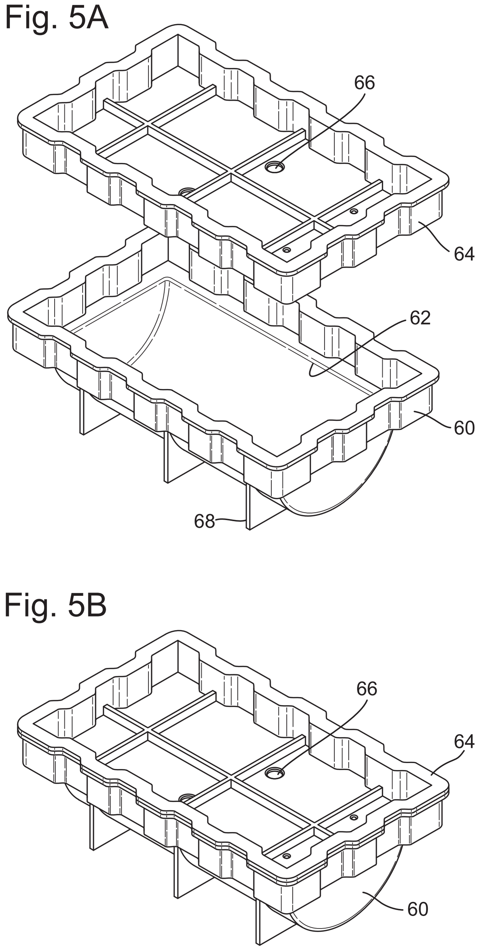

In alternative exemplary embodiments, water may be frozen in two half-cylindrical blocks, which are then placed together in a conduit 20 to form a cylindrical ice pack within an exemplary cold therapy system. In theory, each such solid half-cylindrical ice block could simply be formed using a two-piece mould, such as that illustrated in Figures 5A and 5B of the drawings. A 'bottom' mould 60 includes a half-cylindrical cavity 62 which is covered with a removable 'top' mould 62 that has openings 66 therein, by means of which the cavity 62 in the 'bottom' mould 60 can be filled with water for freezing. Once frozen, the 'top' mould 64 is removed from the 'bottom' mould 60 and the ice block removed from the cavity 62 for use. Base members 68 may extend externally from the arced wall of cavity in the 'bottom' mould 60 to stabilise the unit for filling and transportation for freezing.

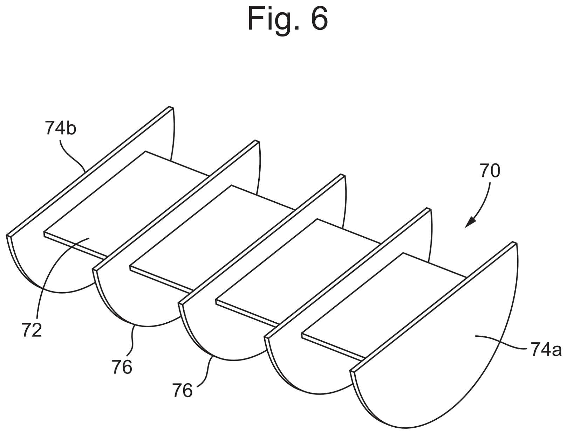

However, in practice, and as illustrated in Figure 6 of the drawings, it is thought to be preferable to use an insert 70 within the cavity 62 of the 'bottom' mould 60, placed therein prior to covering with the 'top' mould 64 and filling with water for freezing. The insert 70, in this example comprises a flat, elongate 'spine' 72 having a semi-circular end plate 74a, 74b disposed at each end thereof. A plurality of semi-circular 'ribs' or spacers 76, similar in size and shape to the end plates 74a, 74b are disposed along the length of the spine 72, in equal spaced-apart relation between the end plates 74a, 74b. The planes of the end plates 74a, 74b and the ribs 76 are parallel to each other and perpendicular to the plane of the spine 72. A small portion of each of the end plates 74a, 74b and the spacers 76 extend beyond the plane of the spine 72 on one side and the remainder thereof, including most of the respective arc, extends beyond the plane of the spine 72 on the opposite side thereof. The arc of each of the end plates 74a, 74b and the spacers 76 substantially matches the arc of the cavity 62 of the 'bottom' mould 60 such that it fits into it with the diametric edges of the end plates 74a, 74b and the spacers 76 extending laterally across the diameter of the cavity 62. The cavity 62 (including therein the insert 70) is then covered with the 'top' mould 64 and filled with water via the openings 66 therein. Once frozen, the insert 70, carrying the ice formed around it within the cavity 62, forms a semi-cylindrical ice pack that can be used in a cold therapy system of the type described above.

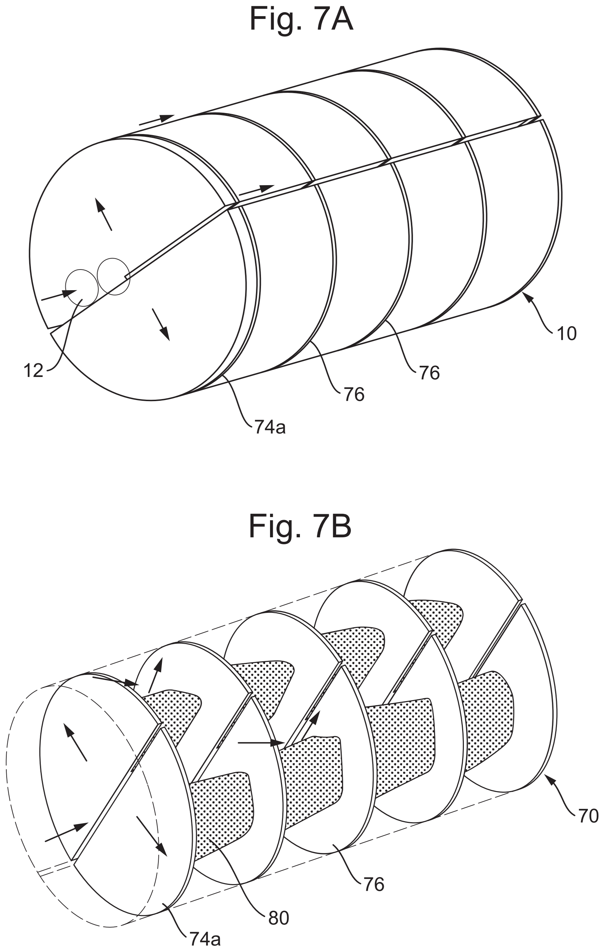

Referring to Figure 7A of the drawings, two of the above-described semi-cylindrical ice packs (including the inserts) are place together (with their diametric edges closest together and parallel to each other) into a conduit 20 of a cold therapy system, for example, of the type described above. It can be seen from Figure 7A, that, during the freezing process described above, ice forms in the cavity 62 such that it covers the end plates 74a, 74b and the spaces between the ribs 76. In use, fluid flows into the container section 16 via the inlet 12 and flows over the ice formed on the proximal end plates 74a of a pair of ice blocks. It also flows all over the outer circumferential area defined by the arcs of the semi-cylindrical ice blocks formed around each insert 70, and also through the space between the diametric edges of the ice blocks, thus maximising the cooling surface area available.

As illustrated in Figure 7B of the drawings, as the ice melts, the configuration of the inserts 70 (i.e. the end plates 74a, 74b and spacers 76) protects the remaining ice 80, thus optimising the time for which the ice blocks can maintain the flow of cooled fluid at the required temperature.

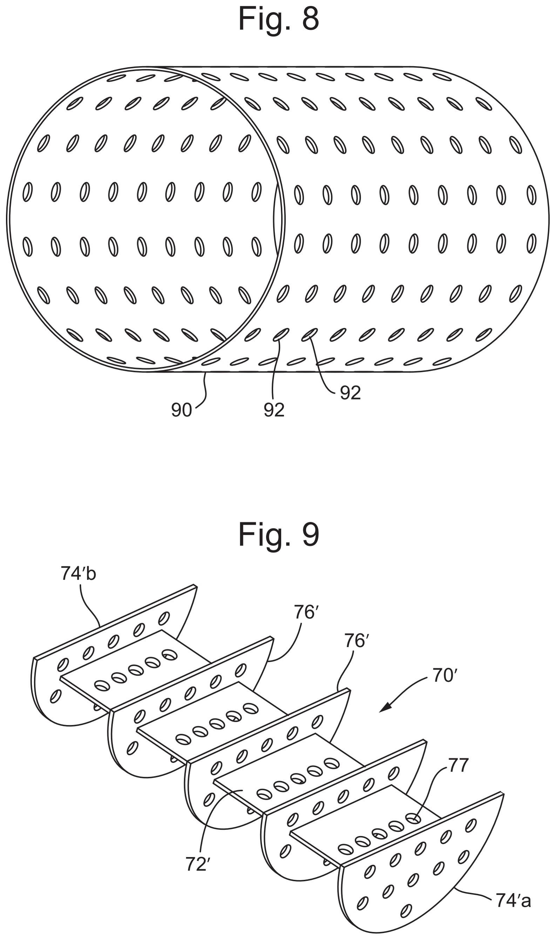

The ice packs described above can be placed directly into the conduits 20 of a cold therapy system. However, alternatively, and referring to Figure 8 of the drawings, a cylindrical sleeve 90 may be provided which can be placed in a conduit 20 before inserting an ice pack. The sleeve 90 may have apertures 92 therein, to further optimise fluid flow over the ice packs.

It will be appreciated by a person skilled in the art, from the foregoing detailed description, that modifications and variations can be made to the described embodiments without departing from the scope of the invention as defined by the appended claims. Different ice pack designs, in particular, are envisaged to optimise management of direct and indirect heat transfer between the fluid flowing into the cold therapy system via the inlet and the solid ice packs. A key object of at least one or more of the examples above is to control the degradation (melting) of the ice to manage cooling of the fluid whilst is flowing over the ice pack(s). Another aim of at least one or more exemplary embodiments is to distribute the fluid flowing over the ice pack(s) so as to optimise heat transfer. These aims and advantages can be achieved with the embodiments described above, but modified and alternative features are also envisaged.

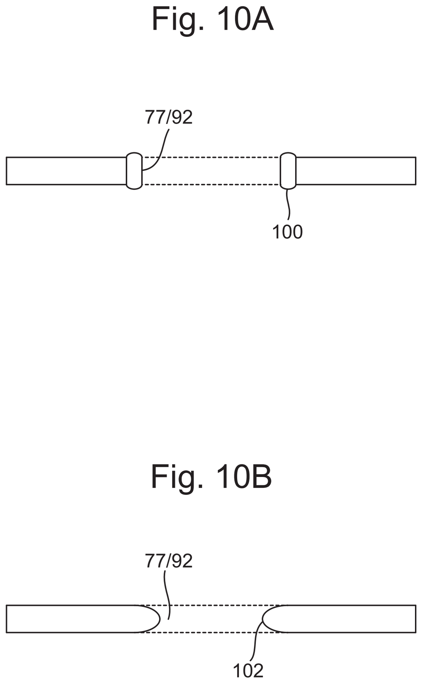

For example, referring to Figure 9 of the drawing, a modified insert 70', for use in making the semi-cylindrical ice blocks, may have a plurality of holes or apertures 77 in the spine 72', the end plates 74'a, 74'b and/or the ribs 76', thus further enabling the surface area of ice over which the fluid flows during cooling to be maximised, especially as the ice block starts to melt. The holes 77 (and/or the holes 92 in the sleeve illustrated in Figure 8 of the drawings) may have lips 100 or ridges, as illustrated schematically in Figure 10A of the drawings, or they may have tapered edges 102, as illustrated schematically in Figure 10B of the drawings. The holes 77 and/or 92 may be of any suitable shape, e.g. circular, oval, slotted, etc., and the present invention is in no way intended to be limited in this regard.

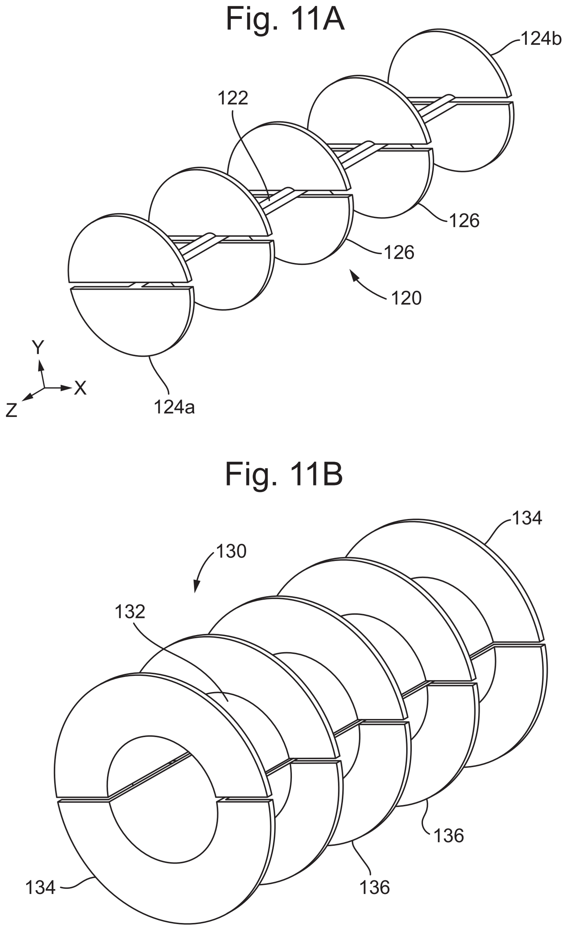

Referring to Figures 11A of the drawings, in yet another exemplary embodiment of the invention, the mould used to form the ice blocks could have a generally cylindrical cavity for receiving an insert 120 comprising an elongate, rigid spine 122 having an endplate 124a, 124b at each end and a number of spacers 126 mounted between the end plates 124a, 124b in equal spaced-apart relation. In this case, the end plates 124a, 124b and spacers 126 are generally circular (to match the shape and configuration of the cavity in the mould) and formed of a pair of semi-circular members held adjacent, but slightly spaced-apart, at their respective diametric edges, by the spine 122. The insert 120 is placed in the mould cavity and the cavity is then filled with water and frozen into a generally cylindrical, solid block of ice around the insert 120. The generally cylindrical ice pack thus formed could be used on its own in a cold therapy system. However, in an alternative embodiment, and referring to Figure 12B of the drawings, the insert 120 of Figure 11A could be configured to fit within a second insert 130. The second insert 130 a tubular spine 132 having a projecting circular collar 134 around the outside of each end of the spine 132 and similarly-shaped spacers 136 in equal spaced-apart relation along the length of the spine between the two collars 134. The tubular spin 132 has an inner diameter suitable to receive the insert of Figure 11A and the assembly comprising the two inserts can be placed in a larger cylindrical mould and filled with water for freezing.

Throughout the specification, the terms "cylindrical" and "semi-cylindrical" are referred to in relation to the ice blocks and packs. Whilst the embodiments illustrate these terms as shapes having parallel side walls and circular or semi-circular cross-sections, the present invention is not necessarily intended to be strictly limited in this regard. The side walls could be tapered, and the cross-sectional area could, alternatively, be oval or any other rounded shape. As such, the terms "cylindrical" and "semi-cylindrical" are to be interpreted broadly and purposively to include such alternative structures. It will also be understood that the invention is not necessarily limited to rounded shapes such as cylinders and, in some embodiments, it is envisaged that the ice packs could, for example, have a square or rectangular cross-section.

Furthermore, the cold water therapy system illustrated in Figures 1A, 1B and 1C of the drawings is described for cooling water for delivery at the outlet. However, it could be adapted to cool alternative fluids, such as air, for example, to cold therapy temperatures for delivery to the user. The materials used to create the ice pack(s) could be varied according to their differing heat transfer properties, so as to deliver different system characteristics and usage. For example, plastics could be used in some embodiments, whereas metals could be used in others, depending on requirements.

## Claims

1. A heat exchange device for use in a cold therapy system comprising a housing having an inlet for receiving fluid from a source and an outlet for delivering cooled fluid, the housing defining a chamber comprising or defining at least one conduit for removably receiving a heat exchange device comprising a body of ice, the conduit being disposed in a fluid flow path between the inlet and the outlet such that, in use, the fluid flows from said inlet over the outer circumference of said body of ice before delivery to said outlet, the heat exchange device comprising a solid ice pack having exposed ice over substantially all of its circumferential area and being configured to fit into said conduit for use.

2. A re-usable mould for making a heat exchange device according to claim 1, the mould comprising a first mould portion defining a cavity therein, and a second mould portion comprising a cover shaped and configured to fit over and cover said cavity, and having one or more through-holes therein to allow said cavity to be filled with water before freezing.

3. A re-usable mould according to claim 2, wherein said cavity has a generally semi-circular or semi-oval profile; and/or further comprising an insert member configured to fit within said cavity, said insert member comprising an elongate spine having at least one external collar or fin thereon, and optionally wherein said insert member is removably mountable within said cavity prior to filling with water.

4. A re-usable mould according to claim 2 or claim 3, comprising a plurality of collars or fins disposed in spaced-apart relation along the length of the spine, each comprising a substantially straight edge and a remaining body portion, a portion of each collar or fin adjacent the respective straight edge extending beyond the surface of the spine on one side and the remaining body portion of each collar or fin extending beyond the surface of the spine on the opposite side thereof; and/or comprising a plurality of symmetrically shaped collars or fins disposed substantially symmetrically thereon and in spaced-apart relation, and optionally wherein each collar or fin comprises of a pair of half plates disposed on opposing sides of the spine to form a respective collar or fin having a central gap between the plates adjacent the spine, the gaps between the plates, collectively, forming an elongate channel through the insert.

5. A re-usable mould according to claim 2 or claim 3, wherein said spine comprises a generally tubular member having a plurality of circumferential fins disposed in spaced-apart relation thereon, and optionally wherein said tubular spine is configured to receive an insert according to claim 8 therein.

6. A re-usable mould according to any of claims 2 to 5, wherein apertures are provided in the walls of the collars or fins and/or the spine.

7. A re-usable mould for making a heat exchange device according to claim 1, comprising a plurality of compartments configured to receive a quantity of water therein for freezing, said compartments being arranged in side-by-side relation and pivotally coupled together at adjacent edges, the mould being configured to be moved, by pivoting said compartments relative to each other, from an open configuration in which said compartments can be filled with water and frozen, and closed configuration in which said compartments form a three-dimensional ice pack having a circumferential side wall of ice.

8. A re-usable mould according to claim 7, wherein said compartments are elongate and of substantially triangular cross-section, and wherein said compartments are pivotally coupled together at adjacent longitudinal edges such that, when in said closed configuration, the apexes of said compartments are located adjacent the centre of the mould and define an elongate channel therebetween that extends along the longitudinal axis of the mould.

9. A re-usable mould according to claim 7, wherein said compartments are elongate and of generally truncated triangular cross-section, and wherein the compartments are pivotally coupled together at adjacent longitudinal edges such that, when in the closed configuration, the truncated apexes of said compartments face the centre of the mould and together form a tubular channel therethrough, and optionally wherein said tubular channel is shaped and configured to receive the re-usable mould of claim 8 when in the closed configuration.

10. A cold therapy system comprising a housing having an inlet for receiving fluid from a source and an outlet for delivering cooled fluid, the housing defining a chamber comprising or defining at least one conduit for removably receiving a heat exchange device comprising a body of ice, the conduit being disposed in a fluid flow path between the inlet and the outlet such that, in use, the fluid flows from said inlet over the outer circumference of said body of ice before delivery to said outlet.

11. A cold therapy system according to claim 10, wherein said heat exchange device comprises an ice pack having a circumferential side wall of exposed ice; or wherein said ice pack comprises a pair of ice packs arranged in said conduit with adjacent edges facing each other and a gap therebetween, and configured such that, in use, fluid flows through said gap.

12. A cold therapy system according to claim 10 or claim 11, further comprising a removable, generally tubular sleeve in said conduit for receiving said ice pack, and optionally wherein said removable sleeve has apertures in its side wall.

13. A cold therapy system according to any of claims 10 to 12, wherein said heat exchange device comprises a plurality of compartments filled with ice, for use, the compartments being arranged relative to each other so as to form a substantially solid ice pack for use.

14. A kit of parts comprising a cold therapy system according to any of claims 10 to 13 and at least one re-usable mould according to any of claims 2 to 9.

15. An ice pack formed using a re-usable mould according to any of claims 2 to 9.
